# EUROPÄISCHE PATENTSCHRIFT

(11) **EP 1 464 633 B1**
(45) Veröffentlichungstag und Bekanntmachung des Hinweises auf die Patenterteilung: **19.09.2007**
(21) Anmeldenummer: 04006755.5
(22) Anmeldetag: 20.03.2004
(51) Int. Cl.: C04B 14/20, C04B 28/24, C04B 28/26

(54) **Verfahren zur Herstellung slikatischer Formkörper**
Process for the production of silicate shaped bodies
Procédé de fabrication des corps faconnés en silicate

(30) Priorität: 02.04.2003 DE 10314977
(43) Veröffentlichungstag der Anmeldung: 06.10.2004
(73) Patentinhaber: H.C. Starck GmbH, 38642 Goslar (DE)
(72) Erfinder: Pantke, Dietrich, Dr., 40882 Ratingen (DE); Schmitz, Peter-Nikolaus, 40764 Langenfeld (DE); Melzer, Hartmut, 40789 Monheim (DE)
(74) Vertreter: Clauswitz, Kai-Uwe Wolfram

(56) Entgegenhaltungen:
- WO-A-97/30951
- DE-A- 19 542 069
- DE-A- 19 851 290
- FR-A- 1 327 505
- US-A- 3 183 115

## Beschreibung

Die Erfindung betrifft ein Verfahren zur Herstellung silikatischer Formkörper.

Silikatische Formkörper haben in großer Bandbreite Eingang in technische Anwendungen gefunden, z.B. als Dämmstoffe im Bausektor, bei Hochtemperaturdichtungen (Asbestersatz) oder zur Formgebung in der Gießereitechnik.

Neben einer kostengünstigen Herstellung haben silikatische Formkörper in nahezu allen Anwendungsgebieten hohe Anforderungen an die mechanische Stabilität zu erfüllen. Zusätzliche Kriterien hängen von den diversen Anwendungen im Einzelnen ab. So ist im Bausektor meist zusätzlich eine hohe Wasserbeständigkeit in Kombination mit einer möglichst geringen Brennbarkeit gefordert; in der Gießereitechnik ist die Feuerfestigkeit das wichtigste Zusatzkriterium.

Die Herstellung der silikatischen Formkörper erfolgt üblicherweise aus silikatischen Rohstoffen, meist natürlichen Ursprungs wie Schichtsilikate oder Sande, und diversen überwiegend anorganischen Bindemitteln.

Als anorganisches Bindemittel werden häufig Wassergläser eingesetzt, die sehr preiswert sind, oft eine hohe mechanische Stabilität der Formkörper erzielen lassen und keine Stoffe enthalten, die zur Brennbarkeit oder Rauchgasentwicklung beitragen. Sie weisen aber den Nachteil hoher Wasserlöslichkeit auf und führen daher zu nicht wasserbeständigen Materialien. Ähnliche Nachteile ergeben sich mit Phosphaten als anorganische Bindemittel, deren Einsatz ebenfalls häufig beschrieben wird.

Aus der Gruppe der anorganischen Bindemittel sind die silikatischen auch deshalb besonders erwünscht, da sie wie die zu bindenden Rohstoffe hauptsächlich aus den umweltverträglichen Siliziumdioxid bestehen und sich diverse Recyclingmöglichkeiten wie z.B. als Füllstoffe im Straßenbau und Landwirtschaft ergeben. Dagegen sind andere anorganische Produkte problematisch beim Recycling und müssen meist nach Gebrauch deponiert werden.

In WO-A 97/30951 wird die Herstellung von Formteilen aus Vermiculiten und anorganischen Bindemitteln wie insbesondere phosphorsäurehaltigen Bindern, Phosphaten oder Wassergläsern beschrieben. Eine gewisse Wasserbeständigkeit kann nur durch den Zusatz organischer Binderkomponenten erreicht werden, der aber unter Brennbarkeitsaspekten begrenzt ist. So sind mit Wasserglas und organischen Komponenten gebundene Platten aus Vermiculiten mit bis zu 10 Gew.-% organischer Komponente bezogen auf das Endprodukt für Dämmstoffsysteme, wie beispielsweise Trennwände für Schiffsladeräume, an die hohe Anforderung im Hinblick auf die Wasserbeständigkeit gestellt werden, nicht geeignet.

DE-A 198 512 90 beschreibt den Einsatz von Wassergläsern und pulverförmiges, ausschließlich anorganisches Bindemittel für Vermiculite bei der Herstellung von nicht brennbaren Platten. Nachteilig ist hier der sehr hohe Alkalisilikatanteil und die entsprechend schlechte Wasserbeständigkeit der Produkte.

Eine Verbesserung der mit Wassergläsern erzielbaren Festigkeiten von Platten auf Basis von Schichtsilikaten gelingt nach US 4 746 555 durch Zusatz von Phenolharzen und Holzspänen unter Inkaufnahme von Problemen bei der Brandlast, der Rauchgasentwicklung und der Entsorgung derartiger Formkörper.

DE-A 195 420 69 beschreibt die Bindung silikatischer Rohstoffe mit einer Mischung aus Wasserglas und pulverförmigem Siliziumdioxid, erreicht aber Wasserbeständigkeit nur durch Behandlung mit einem silikonhaltigen Hydrophobiermittel. Die siliconhaltigen Deckschichten sind zwar wasserabweisend, enthalten aber zum einen immer noch zur Rauchgasentwicklung beitragende organische Anteile und zeigen des Weiteren auch eine geringe mechanische Festigkeit sowie schlechte Bindung zum silikatischen Substrat.

In der WO-A 01/051428 werden hohe mechanische Grünstandfestigkeiten von Dämmstoffplatten durch den Zusatz von organischen Bindern wie Polyurethanen beschrieben. Nach dem Herausbrennen der organischen Bestandteile verbleibt ein rein anorganisches Produkt. Dieser zusätzliche Produktionsschnitt führt jedoch zu höheren Fertigungskosten und umweltbedenklichen Ausbrenngasen. Zudem ist das Endprodukt weiterhin feuchtigkeitsanfällig.

Es besteht daher weiterhin Bedarf an silikatischen Formkörpern, die mechanisch stabil sind und die für die entsprechende Anwendung vorteilhaften zusätzlichen Anforderungen erfüllen. Hier seien beispielsweise hohe Wasserbeständigkeit in Kombination mit einer möglichst geringen Brennbarkeit und Rauchgasentwicklung für die Verwendung im Bausektor oder Feuerfestigkeit für die Anwendung in der Gießereitechnik zu nennen. Auch eine gute Recycelfähigkeit kann von Vorteil sein.

Die Aufgabe bestand daher darin, silikatische Formkörper, die die jeweils geeignete Kombination von Anforderungen erfüllen, und ein Verfahren zu deren Herstellung bereitzustellen.

Überraschend wurde nun gefunden, dass Formkörper, erhältlich nach einem Verfahren bei dem ein silikatischer Feststoff mit einer Lösung enthaltend ein erstes silikatisches Bindemittel vermischt und verpresst wird und anschließend mit einer Lösung enthaltend ein zweites silikatisches Bindemittel nachbehandelt (versiegelt) wird, diese Anforderungen erfüllen.

Die Erfindung betrifft daher ein Verfahren zur Herstellung eines Formkörpers, dadurch gekennzeichnet, dass ein silikatischer Feststoff mit einer Lösung enthaltend ein erstes silikatisches Bindemittel vermischt und die Mischung dann verpresst wird und der gepresste Formkörper anschließend mit einer Lösung enthaltend ein zweites silikatisches Bindemittel nachbehandelt wird.

Der Begriff "silikatisch" ist im Sinne von basierend auf Silikaten, Kieselsäuren oder SiO₂, diese enthaltend oder im Wesentlichen bestehend aus diesen zu verstehen.

Der Begriff "Mischung" kann im Sinne der Erfindung eine Suspension oder Lösung umfassen. Die Mischung kann dickflüssig, zähflüssig, dünnflüssig, solförmig, gelförmig, homogen oder inhomogen sein.

Das erste und zweite silikatische Bindemittel weisen einen unterschiedlichen Modul auf. Der Begriff Modul ist bekannt. Unter Modul in einem silikatischen Bindemittel versteht der Fachmann das analytisch bestimmbare Molverhältnis aus Siliziumdioxid (SiO₂) und Alkalimetalloxid M₂O (M = Lithium, Natrium oder Kalium) im Feststoff des Bindemittels.

Als silikatische Feststoffe sind prinzipiell alle natürlichen oder synthetisch hergestellten Silikate, Kieselsäuren, Kieselsäureformen sowie SiO₂ und dessen spezielle Formen sowie Feststoffe basierend auf diesen Substanzklassen geeignet. Beispielsweise geeignet sind natürliche oder synthetisch hergestellte silikatische Feststoffe wie Kieselsäuren, Pyrokieselsäuren, Quarze, Sande, amorphe oder (teil-)kristalline Alkali- und Erdalkalisilikate oder Alumosilikate (Tonminerale) wie beispielsweise Kaoline, Bentonite, Talkum, Glimmer, Feldspate, Nepheline, Leucite, Olivine, Andalusite, Kyanite, Sillimanite, Mullite, Vermiculite, Perlite, Pumice, Wollastionite, Attapulgite und Sepiolithe, natürliche in Sedimenten vorkommende Zeolithe wie beispielsweise Clinoptilolith, Ereonith und Mordenith sowie Zirkonsilikate. Silikate, deren Systematik und Strukturen sind beispielsweise in F. Liebau: "Die Systematik der Silicate," Naturwissenschaften 49 (1962) 481 -491, in "Silicon" in K. H. Wedepohl (ed.): Handbook of Geochemistry, vol. II/3, chap. 14-A, Springer Verlag, Berlin 1972, pp. 1 - 32 sowie "Classification of Silicates," in P. H. Ribbe (ed.): Orthosilicates, reviews in mineralogy, vol. 5, Min. Soc. Am., 1980, pp. 1- 24 beschrieben.

Bevorzugt werden als silikatischen Feststoffe Schichtsilikate, wie Vermiculite, Perlite oder Glimmer verwendet. Besonders bevorzugt als silikatischer Feststoff ist Vermiculit.

Für Anwendungen, bei denen wie beispielsweise bei Dämmstoffplatten im Bausektor geringe Dichten gefordert sind, werden die Schichtsilikate besonders bevorzugt in ihrer expandierten, d.h. geblähten Form eingesetzt. So kann beispielsweise bevorzugt geblähter Vermiculit mit einer Dichte von 0,08 g/cm³ bis 0,16 g/cm³ eingesetzt werden.

Die silikatischen Feststoffe können im erfindungsgemäßen Verfahren in unterschiedlichen Korngrößen eingesetzt werden. So können beispielsweise Vermiculite in Pulverform oder als Granulat eingesetzt werden, wobei die mittleren Teilchengrößen beispielsweise bei 1 µm und 30 mm, bevorzugt bei 1 µm und 10 mm Durchmesser liegen können. In bevorzugten Ausführungsformen werden handelsübliche Vermiculite eingesetzt.

Die silikatischen Feststoffe werden bevorzugt mit einer Lösung enthaltend ein erstes silikatisches Bindemittel mit einem Modul von höchstens 50, besonders bevorzugt von 1,5 bis 15, ganz besonders bevorzugt von 1,5 bis 10 vermischt.

Als Lösungsmittel für die silikatischen Bindemittel eignen sich prinzipiell alle Lösungsmittel, die keine Ausfällung des silikatischen Bindemittels bzw. keine für das erfindungsgemäße Verfahren nachteilige Veränderung des Bindemittels bewirken. Bevorzugt eignen sich als Lösungsmittel für die silikatischen Bindemittel Wasser oder Alkohole, beispielsweise Methanol, Ethanol, Propanol, Isopropanol und höhere Homologe, sowie Mischungen aus diesen in beliebigen Mischungsverhältnissen. Besonders bevorzugt eignet sich Wasser als Lösungsmittel für die silikatischen Bindemittel.

Unter Lösungen enthaltend silikatische Bindemittel sind im Sinne der Erfindung auch stabile Suspensionen, Sole oder Gele sowie kolloidale bzw. kolloiddisperse Lösungen, wie beispielsweise Kieselsäuresole (Kieselsole), zu verstehen. Kieselsole und deren Herstellung sind dem Fachmann bekannt. Kieselsole sind kommerziell erhältlich, beispielhaft seien hier die unter dem Handelsnamen Lewasil^{®} erhältlichen Produkte der Fa. H. C. Starck GmbH genannt.

Kieselsäuresole sind kolloidale Lösungen von amorphem Siliciumdioxid in Wasser, die auch als Siliciumdioxidsole meist aber kurz als Kieselsole bezeichnet werden. Das Siliciumdioxid liegt dabei in Form von kugelförmigen und an der Oberfläche hydroxilierten Partikeln vor. Der Partikeldurchmesser der Kolloidteilchen beträgt in der Regel 1 bis 200 nm, wobei die zur Teilchengröße korrelierende spezifische BET-Oberfläche (bestimmt nach der Methode von G.N.Sears, Analytical Chemistry Vol. 28, N. 12, 1981-1983, Dezember 1956) bei 15 bis 2000 m²/g liegt. Die Oberfläche der SiO₂-Teilchen weist eine Ladung auf, die durch ein entsprechendes Gegenion ausgeglichen wird, das zur Stabilisierung der kolloidalen Lösung führt.

Häufig sind Kieselsole anionisch und alkalisch stabilisiert. Solche Kieselsole besitzen einen pH-Wert von 7 bis 11,5 und enthalten als Alkalisierungsmittel beispielsweise geringe Mengen Na₂O, K₂O, Li₂O, Ammoniak, organische Stickstoffbasen, Tetraalkylammoniumhydroxide oder Alkali- oder Ammoniumaluminate. Anionische Kieselsole können auch als semistabile kolloidale Lösungen schwach sauer vorliegen.

Ferner ist es möglich, durch Beschichtung der Oberfläche mit geeigneten Salzen wie beispielsweise Al₂(OH)₅Cl Kieselsole mit kationisch geladenen Partikeln herzustellen.

Der Herstellprozess für Kieselsole durchläuft im wesentlichen die Produktionsschritte Entalkalisierung von Wasserglas mittels Ionenaustausch, Einstellung und Stabilisierung der jeweils gewünschten Teilchengrößen(verteilung) der SiO₂-Partikel, Einstellung der jeweils gewünschten SiO₂-Konzentration und gegebenenfalls einer Oberflächenmodifikation der SiO₂-Partikel, wie beispielsweise mit Al₂(OH)₅Cl. In keinem dieser Schritte verlassen die SiO₂-Partikel den kolloidal gelösten Zustand. Dadurch erklärt sich das Vorliegen der diskreten Primärpartikel mit beispielsweise hoher Bindereffektivität.

Als silikatische Bindemittel im Sinne der Erfindung kommen beispielsweise lösliche Alkalisilikate, Kieselsäuren, SiO₂ in beliebigen Modifikationen und Formen und Mischungen aus diesen in Frage. Beispielhaft seien hier Wassergläser, wie beispielsweise die kommerziell erhältlichen Natron- oder Kaliwassergläser, und nicht modifizierte sowie modifizierte Kieselsole, vorzugsweise die kommerziell erhältlichen, aufgeführt.

In bevorzugten Ausführungsformen des erfindungsgemäßen Verfahrens wird als Lösung eines ersten silikatischen Bindemittels Wasserglas gegebenenfalls im Gemisch mit Siliziumdioxid, bevorzugt in Form von Kieselsol, und gegebenenfalls weiterem zusätzlichem Lösungsmittel eingesetzt.

Die Lösungen enthaltend silikatische Bindemittel sind nach dem Fachmann bekannten Verfahren herstellbar. Beispielsweise kann eine geeignete Lösung enthaltend ein silikatisches Bindemittels durch Vermischen von Alkalisilikaten oder Lösungen von Alkalisalzen, z.B. Wasserglas, wie beispielsweise Natron- oder Kaliwasserglas, oder anderen wasserlöslichen Alkalisilikaten oder deren Lösungen, mit Siliziumdioxid und gegebenenfalls Zugabe von zusätzlichem Lösungsmittel gegebenenfalls unter anschließender Nachbehandlung dieser Lösung, z.B. durch Rühren gegebenenfalls unter Erhitzen, hergestellt werden. Das Mischungsverhältnis Alkalisilikat und Siliziumdioxid wird zur Herstellung des ersten Bindemittels so gewählt, dass dieses ein Modul von höchstens 50, bevorzugt 1,5 bis 15, ganz besonders bevorzugt von 1,5 bis 10 aufweist, und zur Herstellung des zweiten Bindemittels so gewählt, dass dieses ein Modul von mindestens 10, bevorzugt 20 bis 1000, besonders bevorzugt 50 bis 200 aufweist.

Das Siliziumdioxid wird dabei beispielsweise in Form von Fällungskieselsäuren, pyrogenen Kieselsäuren oder Kieselsolen eingesetzt. Vorzugsweise wird es in Form von Kieselsol eingesetzt.

Die Lösung enthaltend ein erstes silikatisches Bindemittel weist bevorzugt einen Feststoffanteil von 5 bis 60 Gew.-%, vorzugsweise 20 bis 55 Gew.-% bezogen auf das Gesamtgewicht der Lösung auf.

Die Mischung aus zu bindendem silikatischem Feststoff und der Lösung enthaltend ein erstes silikatisches Bindemittel enthält das erste silikatische Bindemittel in einer solchen Menge, dass der Feststoffanteil des silikatischen Bindemittels 1 bis 20 Gew.-%, bevorzugt 2 bis 15 Gew.-%, besonders bevorzugt 4 bis 12 Gew.-% bezogen auf das Gewicht des resultierenden Formkörpers vor der Nachbehandlung entspricht. In bevorzugten Ausführungsformen, in denen als silikatischer Feststoff ein solcher mit hoher Saugfähigkeit, wie beispielsweise ein Vermikulit mit hohem Blähgrad und somit geringer Dichte eingesetzt wird, kann die Mischung aus zu bindendem silikatischem Feststoff und der Lösung enthaltend ein erstes silikatisches Bindemittel das erste silikatische Bindemittel in einer solchen Menge enthalten, dass der Feststoffanteil des silikatischen Bindemittels 1 bis 50 Gew.-%, bevorzugt 2 bis 35 Gew.-%, besonders bevorzugt 4 bis 25 Gew.-% bezogen auf das Gewicht des resultierenden Formkörpers vor der Nachbehandlung entspricht.

Das Vermischen der Lösung enthaltend das erste silikatische Bindemittel mit dem silikatischen Feststoff kann beispielsweise mit Hilfe von geeigneten Maschinen, wie beispielsweise Schaufelmischem, durchgeführt werden.

Die Mischung aus silikatischem Feststoff und der Lösung enthaltend das erste silikatische Bindemittel wird bei 20 bis 200°C, bevorzugt 20 bis 120°C, verpresst. Das Verpressen kann kontinuierlich oder diskontinuierlich erfolgen und kann nach den üblichen, dem Fachmann bekannten Verfahren durchgeführt werden. Beispielsweise können die bei der Spanplattenherstellung eingeführten Pressmaschinen nach den entsprechenden Verfahren eingesetzt werden, wie beispielsweise in Ullmann's Encyclopedia of Industrial Chemisty (1996) Vol.A28, Seite 331 bis 333 beschrieben.

Zur Erzielung einer ausreichenden Anfangsfestigkeit des gepressten Formkörpers vor der Nachbehandlung reicht bereits ein minimaler Pressdruck aus. Eine ausreichende Anfangsfestigkeit ist dann gegeben, wenn der gepresste Formkörper durch die anschließende Nachbehandlung nicht beschädigt oder sogar zerstört wird, d.h. beispielsweise bei Tauchen oder Tränken mit dem zweiten silikatischen Bindemittel nicht auseinander bricht oder vollständig zerfällt. In bevorzugten Ausführungsformen des erfindungsgemäßen Verfahrens werden Pressdrücke von mindestens 2 kg/cm² angewandt. In weiteren bevorzugten Ausführungsformen können jedoch auch kleinere Pressdrücke als 2 kg/cm² angewandt werden.

Der so erhaltene lösungsmittelhaltige Formkörper kann nach dem Pressvorgang und vor der Nachbehandlung getrocknet werden. Diese Zwischentrocknung kann bei Temperaturen von 10 bis 200°C, bevorzugt 20 bis 150°C erfolgen. So ist beispielsweise eine Trocknung bei Raumtemperatur ebenso wie eine Trocknung bei erhöhter Temperatur z.B. in entsprechenden Trockenschränken, Trockenräumen oder Öfen möglich. Auch eine Trocknung mittels Gefriertrocknung ist denkbar, sofern der gepresste Formkörper dabei nicht beschädigt wird. In bevorzugten Ausführungsformen wird der gepresste Formkörper vor der Nachbehandlung getrocknet. Dies ist insbesondere dann von Vorteil, wenn die Festigkeit des gepressten Formkörpers vor der Nachbehandlung erhöht werden soll. So kann ebenfalls eine ausreichende Anfangsfestigkeit für die anschließende Nachbehandlung erzielt werden. Je nach Trocknungstemperatur und gewünschtem Trocknungsgrad kann die Trocknungszeit entsprechend variieren.

In einer bevorzugten Ausführungsform kann die Anfangsfestigkeit auch durch Begasen mit CO₂ gesteigert werden. Dieses Verfahren ist beispielsweise von der Kemsandbindung mit Wassergläsern in der Gießereitechnik bekannt.

Nach dem Verpressen erfolgt erfindungsgemäß die Nachbehandlung (Versiegelung) des so erhaltenen Formkörpers mit einer Lösung enthaltend ein zweites silikatisches Bindemittel (Versiegelungsmittel).

Das zweite silikatische Bindemittel weist einen gegenüber dem ersten silikatischen Bindemittel höheren Modul auf. Der Modul des zweiten silikatischen Bindemittels ist bevorzugt mindestens 10, besonders bevorzugt 20 bis 1000, ganz besonders bevorzugt 50 bis 200.

Die Lösung enthaltend das zweite silikatische Bindemittel weist bevorzugt einen Feststoffanteil von 5 bis 60 Gew.-%, besonders bevorzugt 20 bis 55 Gew.-% bezogen auf das Gesamtgewicht der Lösung auf.

In bevorzugten Ausführungsformen des erfindungsgemäßen Verfahrens wird als Lösung eines zweiten silikatischen Bindemittels ein Kieselsol gegebenenfalls im Gemisch mit Wasserglas und gegebenenfalls weiterem zusätzlichem Lösungsmittel eingesetzt.

Die Nachbehandlung kann durch Tauchen, Tränken, Aufsprühen oder Aufstreichen und anschließender Trocknung gegebenenfalls unter Druck erfolgen. Die Dauer der Nachbehandlung richtet sich nach dem Verfahren. Bei einem Tauch- oder Tränkprozess kann beispielsweise bereits nach wenigen Sekunden eine ausreichende Aufnahme an Binderflüssigkeit erreicht werden. Es sind aber auch längere Nachbehandlungszeiten von bis zu mehreren Stunden möglich. Die Trocknung kann ebenso wie die oben bereits beschriebene Zwischentrocknung des vor der Nachbehandlung gepressten Formkörpers bei Temperaturen von 10 bis 200°C, bevorzugt 20 bis 150°C erfolgen. Sie kann ebenfalls beispielsweise bei Raumtemperatur ebenso wie bei erhöhter Temperatur z.B. in entsprechenden Trockenschränken, Trockenräumen oder Öfen durchgeführt werden. Auch eine Trocknung mittels Gefriertrocknung ist denkbar, sofern der nachbehandelte Formkörper dabei nicht beschädigt wird. Je nach Trocknungstemperatur kann die Trocknungszeit entsprechend variieren. Dabei sind Trocknungszeiten von wenigen Minuten oder Stunden bis hin zu mehreren Tagen oder sogar Wochen möglich.

Im Vergleich zum Feststoffanteil des Formkörpers an erstem silikatischen Bindemittel nach dem Verpressen wird durch die Nachbehandlung der Gesamtfeststoffanteil an erstem und zweitem silikatischen Bindemittels auf 2 bis 40 Gew.-%, bevorzugt auf 5 bis 30 Gew.-%, besonders bevorzugt auf 8 bis 25 Gew.-% bezogen auf das Gewicht des nach der Nachbehandlung resultierenden Formkörpers erhöht. In bevorzugten Ausführungsformen, in denen als silikatischer Feststoff ein solcher mit hoher Saugfähigkeit, wie beispielsweise ein Vermikulit mit hohem Blähgrad und somit geringer Dichte eingesetzt wird, kann - im Vergleich zum Feststoffanteil des Formkörpers an erstem silikatischen Bindemittel nach dem Verpressen - durch die Nachbehandlung der Gesamtfeststoffanteil an erstem und zweitem silikatischen Bindemittels auf 2 bis 60 Gew.-%, bevorzugt auf 5 bis 45 Gew.-%, besonders bevorzugt auf 8 bis 35 Gew.-% bezogen auf das Gewicht des nach der Nachbehandlung resultierenden Formkörpers erhöht werden.

Daher sind erfindungsgemäß hergestellte Formkörper, dadurch gekennzeichnet, dass sie einen Gesamtanteil an silikatischen Bindemitteln von 2 bis 40 Gew.-%, bevorzugt 5 bis 30 Gew.-%, besonders bevorzugt 8 bis 25 Gew.-% bezogen auf das Gesamtgewicht des Formkörpers und einen Anteil an silikatischem Feststoff von 60 bis 98 Gew.-%, bevorzugt 70 bis 95 Gew.-%, besonders bevorzugt 75 bis 92 Gew.-% bezogen auf das Gesamtgewicht des Formkörpers enthalten.

Erfindungsgemäß hergestellte Formkörper sind weiterhin solche, die im Wesentlichen aus silikatischen Bestandteilen, d.h. silikatischem Feststoff und silikatischem Bindemittel bzw. gegebenenfalls Reaktionsprodukten aus diesen, die durch Zusammenführen der Komponenten und/oder bedingt durch das Herstellverfahren des Formkörpers, z.B. Temperaturbehandlung, Verpressen etc., entstanden sind, bestehen. Bevorzugt sind dies solche Formkörper die weniger als 2 Gew.-%, bevorzugt weniger als 1 Gew.-%, besonders bevorzugt weniger als 0,1 Gew.-% organische Komponenten, bezogen auf das Gesamtgewicht des Formkörpers, enthalten. Besonders bevorzugt sind die erfindungsgemäß hergestellten Formkörper solche, die einen Gesamtanteil an silikatischen Bindemitteln von 2 bis 40 Gew.-%, bevorzugt 5 bis 30 Gew.-%, besonders bevorzugt 8 bis 25 Gew.-%, bezogen auf das Gesamtgewicht des Formkörpers und einen Anteil an silikatischem Feststoff von 60 bis 98 Gew.-%, bevorzugt 70 bis 95 Gew.-%, besonders bevorzugt 75 bis 92 Gew.-% bezogen auf das Gesamtgewicht des Formkörpers enthalten, mit der Maßgabe, dass die Summe der beiden Anteile 100 Gew.-% ergibt.

Die erfindungsgemäß hergestellten Formkörper weisen im bevorzugten Ausführungsformen einen Alkaligradienten auf, dahingehend dass die äußere Schicht des Formkörpers einen geringeren Anteil an Alkaliionen in ihrer Zusammensetzung aufweist als das Innere des Formkörpers.

Durch die erfindungsgemäße Nachbehandlung mit der Lösung eines zweiten Bindemittels wird die mechanische Festigkeit des nach dem erfindungsgemäßen Verfahren hergestellten Formkörpers im Vergleich zu der nach dem Verpressen erreichten gesteigert und eine Festigkeit erzielt, die allein durch Erhöhung des Feststoffanteils des ersten silikatischen Bindemittels im ersten Bindeschritt nicht möglich ist. Die erfindungsgemäße Nachbehandlung mit der Lösung eines zweiten Bindemittels führt darüber hinaus zu einer höheren Wasserbeständigkeit des nach dem erfindungsgemäßen Verfahren hergestellten Formkörpers. Diese vorteilhaften Eigenschaften, insbesondere die erhöhte Wasserbeständigkeit, der nach dem erfindungsgemäßen Verfahren erhältlichen Formkörper werden ohne Beimischung von organischen Komponenten, wie organischen Bindemitteln oder sonstigen organischen Zusatzstoffen, die in bekannten Formkörpern entweder verbleiben, wie z.B. in US 4 746 555 beschrieben, oder aber nachträglich durch aufwendige Verfahren aus diesen entfernt werden müssen, wie z.B. in WO-A 01/051428 beschrieben, erzielt.

Des Weiteren sind die nach dem erfindungsgemäßen Verfahren erhältlichen Formkörper nicht brennbar und entwickeln im Brandfall keine Rauchgase.

Durch Verwendung von blähfähigen silikatischen Feststoffen wie z.B. Vermiculiten, Perliten oder Glimmer, vorzugsweise Vermiculiten, können poröse Formkörper erhalten werden, die auch bei einer geringen Dichte sehr gute mechanische Festigkeiten aufweisen. Die porösen silikatischen Formkörper weisen in der Regel Dichten von 0,2 bis 2,0 g/cm³, bevorzugt 0,3 bis 1,5 g/cm³ und besonders bevorzugt 0,4 bis 1,2 g/cm³ auf.

Nach dem erfindungsgemäßen Verfahren erhältliche Formkörper können beispielsweise Platten, Blöcke, Schalen, Röhren oder Halbschalen sein. Die nach dem erfindungsgemäßen Verfahren erhältlichen Formkörper können für vielfältige Zwecke als Konstruktions-, Dämm-, Hochtemperaturdichtungs- oder Feuerfestmaterial verwendet werden. Bevorzugt werden die erfindungsgemäß hergestellten Formkörper als Trägerplatten für diverse Dämmstoffmaterialien wie z.B. Polystyrolblöcke, Polyurethanblöcke, oder Mineralwollmatten (z.B. Steinwolle) verwendet. Besonders vorteilhaft ist auch, dass sich Oberfläche der nach dem erfindungsgemäßen Verfahren erhältlichen Formkörper in besonderem Maße zur Verklebung mit anderen im Dämmstoffbereich üblichen Materialien wie z.B. Metallen bzw. Metallfolien eignet.

Weiterhin werden die nach dem erfindungsgemäßen Verfahren erhältlichen Formkörper als Dämmstoffe mit thermischer Isolationswirkung, und als Feuerfestmaterialien verwendet.

Es ist ebenfalls möglich, die nach dem erfindungsgemäßen Verfahren erhältlichen Formkörper in der Gießereitechnik und als Hochtemperaturdichtungen einzusetzen.

Die im Folgenden aufgeführten Beispiele dienen der Erläuterung der Erfindung und sind nicht als Beschränkung aufzufassen.

### Beispiele

### Beispiel 1:

### Herstellung einer Lösung enthaltend ein erstes silikatisches Bindemittel mit Modul 6

Zur Herstellung einer Lösung enthaltend ein silikatisches Bindemittel mit Modul 6 werden 56,1 g Kaliwasserglas (Kaliwasserglas 35, Cognis Deutschland GmbH) mit 19,9 g 50 %igem Kieselsol (Levasil^{®} 50/50 %, H.C.Starck GmbH) und 22,4 g deionisiertem Wasser eine Minute bei 20°C mit einem Magnetrührer gemischt. Man erhält eine Lösung mit 30,0 Gew.-% Feststoffgehalt an silikatischem Bindemittel.

### Beispiel 2:

### Herstellung einer Lösung enthaltend ein zweites silikatisches Bindemittel

Zur Herstellung einer Lösung enthaltend ein silikatisches Bindemittel mit Modul 50 werden 2,8 g Kaliwasserglas (Kaliwasserglas 35, Cognis Deutschland GmbH) mit 19,9 g 50 %igem Kieselsol (Levasil^{®} 50/50 %, H.C.Starck GmbH) und 14,0 g deionisiertem Wasser wie unter Beispiel 1 angegeben vermischt. Man erhält eine Lösung mit 30,0 Gew.-% Feststoffgehalt an silikatischem Bindemittel.

### Beispiel 3:

### Erfindungsgemäße Herstellung eines Formkörpers aus Vermiculit mit einem ersten Binder mit Modul 6 und einem zweiten mit Modul 50

100 Gewichtsteile eines Vermiculites mit einer mittleren Korngröße von ca. 1 mm (Vermiculit SFX, Micronized Products (PTY) Ltd) werden mit 20 Gewichtsteilen einer Lösung enthaltend ein erstes silikatisches Bindemittel gemäß Beispiel 1 (einer wässrigen Alkalisilikatlösung mit einem Modul von 6 und einem Feststoffgehalt von 30 Gew.-%) mit einer Küchenmaschine (Braun Multipraktic 120, Einsatz: Rührbesen) vermischt. Nach 5 Minuten wird eine ausreichende Homogenisierung erreicht.

Von der so erhaltenen Masse werden 2,2 g bei 20°C in einer Stempelpresse verpresst. Der Stempeldurchmesser beträgt 20 mm, der Pressweg 50 mm und das Auflagegewicht 22,3 kg. Damit resultiert ein Druck von 7,1 kg/cm².

Der erhaltene Pressling (gepresster Formkörper) wird bei 110°C bis zur Gewichtskonstanz getrocknet.

Der so erhaltene Formkörper wird 5 Sekunden in der Lösung enthaltend ein zweites silikatisches Bindemittel gemäß Beispiel 2 (eine wässrige Alkalisilikatlösung mit einem Modul von 50 und einem Feststoffgehalt von 30 Gew.-%) getränkt.

Abschließend wird der resultierende Formkörper ebenfalls bei 110°C bis zur Gewichtskonstanz getrocknet.

Es wird ein erfindungsgemäß hergestellter Formkörper von 20 mm Durchmesser, von 10 mm Dicke (Tablettenform) und mit einer Dichte von 0,9 g/cm³ erhalten. Der Bindergesamtanteil bezogen auf das Gesamtgewicht des Formkörpers liegt bei 23 Gew.-%.

Der Formkörper weist eine sehr gute mechanische Festigkeit bei gleichzeitig sehr guter Wasserbeständigkeit auf, er ist nicht brennbar und entwickelt selbst beim Glühen keine Rauchgase.

Die Bestimmung der mechanischen Festigkeit erfolgt mit einem Tablettenhärtetester der Firma Schleuniger, Modell 2E/20S. Die maximale Krafteinwirkung beträgt 20 kp. Die Tabletten werden liegend in der Apparatur eingespannt.

Im vorliegenden Fall von Beispiel 3 wurden 17 kp erreicht.

Die Beurteilung der Brennbarkeit und der Rauchgasentwicklung erfolgt optisch nach einminütigem Kontakt mit einer offenen Flamme eines handelsüblichen Gaskartuschenbrenners. Bei den Tests betrug die Flammentemperatur 900-1000°C.

Beim Formkörper nach Beispiel 3 wurde keine Brennbarkeit und keine Rauchgasentwicklung gefunden.

Zur Überprüfung der Wasserbeständigkeit wird jeweils ein Formkörper in 100 ml Wasser in einer geschlossenen Flasche gelagert. Dabei wird als Kriterium die Zeitspanne bis zum ersten visuell erkennbaren Ablösen von Partikeln bis hin zum vollständigen Zerfall des Formkörpers ermittelt.

Beim Formkörper nach Beispiel 3 lösten sich erste Partikel nach 14 Tagen, der vollständige Zerfall trat nach 100 Tagen ein.

### Beispiel 4:

Erfindungsgemäße Herstellung eines Formkörpers aus Vermiculit mit 35 %igem Kaliwasserglas (Cognis Deutschland GmbH) als erstem und Levasil^{®} 300/30% (H.C.Starck GmbH) als zweitem Binder

100 Gewichtsteile Vermiculit SFX werden mit 20 Gewichtsteilen Kaliwasserglas 35 gemäß der Vorgehensweise in Beispiel 3 gemischt, gepresst, getrocknet, in Levasil^{®} 300/30% getränkt und endgetrocknet.

Es wird ein Formkörper (18 Gew.-% Binderanteil bezogen auf das Gesamtgewicht des Formkörpers bei sonst gleichen Maßen wie in Beispiel 3) mit sehr guter mechanischer Festigkeit (> 20 kp) und sehr guter Wasserbeständigkeit (erste Partikelablösungen nach 30 Tagen, vollständiger Zerfall > 100 Tagen). Der Formkörper ist nicht brennbar und entwickelt keine Rauchgase.

### Beispiel 5:

### Herstellung eines hochkonzentrierten Kaliwasserglases mit ca. 55 % Feststoffgehalt

In einem 6 Liter-Dreihalskolben werden 2500 g Kaliumhydroxid (plätzchenform, > 85 %) und 430 g Wasser gemischt und unter Kühlung und starkem Rühren mit 5130 g Levasil^{®} 50/50% (H.C.Starck GmbH) versetzt.

Nach 3 h erhält man ein Kaliwasserglas mit ca. 55 % Feststoffgehalt und einem Modul von ca. 2,3.

### Beispiel 6:

Erfindungsgemäße Herstellung eines Formkörpers aus Vermiculit mit hochkonzentriertem Kaliwasserglas als erstem und Levasil^{®} 300/30% als zweitem Binder

Nach einer Vorgehensweise gemäß den Beispielen 3 und 4 wird ein Formkörper (19 Gew.-% Binderanteil bezogen auf das Gesamtgewicht des Formkörpers) mit sehr hoher mechanischer Festigkeit (> 20 kp) und sehr guter Wasserbeständigkeit (erste Partikelablösung nach 100 Tagen, endgültiger Zerfall > 100 Tage) erhalten. Der Formkörper ist nicht brennbar und entwickelt keine Rauchgase.

### Beispiel 7:

### Herstellung einer Lösung enthaltend ein erstes silikatisches Bindemittel mit Modul 10

Zur Herstellung einer Lösung enthaltend ein silikatisches Bindemittel mit Modul 10 werden 57,0g Kaliwasserglas (Kaliwasserglas 35, Cognis Deutschland GmbH) mit 99,9 g 30 %igem Kieselsol (Levasil^{®} 300/30 %, H.C.Starck GmbH) eine Minute bei 20°C mit einem Magnetrührer gemischt. Man erhält eine Lösung mit 33,0 Gew.-% Feststoffgehalt an silikatischem Bindemittel.

### Beispiel 8:

Erfindungsgemäße Herstellung eines Formkörpers aus Mica mit einem silikatischen Binder mit Modul 10 als erstem und Levasil^{®} 300/30% als zweitem Binder

60 Gewichtsteile Mica mit geringer Korngröße (60 mesh, Micronized Produkts Pty) werden mit 25 Gewichtsteilen einer Lösung enthaltend ein erstes silikatisches Bindemittel gemäß Beispiel 7 (einer wässrigen Alkalisilikatlösung mit einem Modul von 10 und einem Feststoffgehalt von 33 Gew.-%) gemäß der Vorgehensweise in Beispiel 3 gemischt, gepresst, getrocknet, in Levasil^{®} 300/30% getränkt und endgetrocknet.

Es wird ein Formkörper (40 Gew.-% Binderanteil bezogen auf das Gesamtgewicht des Formkörpers bei sonst gleichen Maßen wie in Beispiel 3) mit sehr guter mechanischer Festigkeit (> 20 kp) und sehr guter Wasserbeständigkeit (erste Partikelablösungen nach 50 Tagen, vollständiger Zerfall > 100 Tagen) erhalten. Der Formkörper ist nicht brennbar und entwickelt keine Rauchgase.

### Vergleichsversuche

Zu Vergleichzwecken werden Formkörper aus Vermiculit mit Kaliwasserglas 35 oder Natronwasserglas 37/40 (Cognis Deutschland GmbH) sowie deren Mischungen mit bis zu 10 Gew.-% an handelsüblichen Polyacrylaten, wie Mowilith DM 60 (Celanese AG), als organischer Binderkomponente ohne Nachbehandlung hergestellt.

Bei den einstufigen Vergleichsversuchen werden Formkörper mit deutlichen Nachteilen bei Festigkeit, im Folgenden auch als mechanische Festigkeit bezeichnet, Wasserbeständigkeit, Brennbarkeit und/oder Rauchgasentwicklung erhalten.

### Vergleichsbeispiel 1: Herstellung eines Formkörpers aus Vermiculit mit Kaliwasserglas als Bindemittel ohne Nachbehandlung

100 Gewichtsteile eines Vermiculites (Typ wie bei Beispiel 3) werden mit 20 Gewichtsteilen Kaliwasserglas 35 (Cognis Deutschland GmbH, 35 % Feststoffgehalt, Modul ca. 3,4) wie bei Beispiel 3 vermischt. Pressung und Trocknung erfolgen ebenfalls wie bei Beispiel 3 beschrieben. Es erfolgt keine Nachbehandlung.

Es wird ein Formkörper (6,5 Gew.-% Bindemittelanteil bezogen auf das Gesamtgewicht des Formkörpers bei sonst gleichen Maßen wie in Beispiel 3) mit guter mechanischer Festigkeit (12 kp) erhalten, der zwar nicht brennbar ist und keine Rauchgase entwickelt, allerdings eine sehr schlechte Wasserbeständigkeit (erste Partikelablösungen nach 2 Tagen, vollständiger Zerfall nach 7 Tagen) aufweist.

### Vergleichsbeispiel 2: Herstellung eines Formkörpers aus Vermiculit mit einer Wasserglas/Polyacrylat-Mischung als Bindemittel ohne Nachbehandlung

100 Gewichtsteile eines Vermiculites (Typ wie bei Beispiel 3) werden mit 40 Gewichtsteilen einer 1:1 Mischung aus Kaliwasserglas 35 und Mowilith DM 60 vermischt. Das weitere Verfahren erfolgt gemäß Vergleichsbeispiel 1.

Es wird ein Formkörper (6,5 Gew.-% anorganischer und 10 Gew.-% organischer Bindemittelanteil bezogen auf das Gesamtgewicht des Formkörpers bei sonst gleichen Maßen wie in Beispiel 3) mit guter mechanischer Festigkeit (> 20 kp) erhalten. Der Formkörper ist zwar nur gering brennbar, entwickelt aber Rauchgase und zeigt zudem eine schlechte Wasserbeständigkeit (erste Partikelablösungen nach 3 Tagen, vollständiger Zerfall nach 35 Tagen).

Die erfindungsgemäß hergestellten Vermiculit-Formkörper aus den Beispielen 3, 4 und 6 zeigen gegenüber den in den Vergleichsbeispielen 1 und 2 ohne Nachbehandlung hergestellten Formkörpern zum einen höhere mechanische Festigkeit und höhere Wasserbeständigkeit, zum anderen sind sie nicht brennbar und entwickeln keine Rauchgase.

### Vergleichsbeispiel 3: Herstellung eines Formkörpers aus Vermiculit mit Kaliwasserglas 35 als erstem und zweitem Bindemittel

Zu weiteren Vergleichszwecken wird ein Formkörper aus Vermiculit mit Kali- oder Natronwasserglas als erstem und zweitem Bindemittel hergestellt.

Nach einer Vorgehensweise gemäß den Beispielen 3, 4 und 6 ein Formkörper (23 Gew.-% Binderanteil bezogen auf das Gesamtgewicht des Formkörpers) mit sehr hoher mechanischer Festigkeit (> 20 kp) erhalten. Der Formkörper ist nicht brennbar und entwickelt keine Rauchgase. Er weist aber eine schlechte Wasserbeständigkeit auf (erste Partikelablösung nach 3 Tagen, vollständiger Zerfall nach ca. 50 Tagen) auf. Zudem treten Verbackungen zum Presswerkzeug auf.

Die erfindungsgemäß hergestellten Vermiculit-Formkörper aus den Beispielen 3, 4 und 6 zeigen gegenüber dem nur mit Kaliwasserglas gebundenen Formkörper aus Vergleichsbeispiel 3 eine deutlich bessere Wasserbeständigkeit bei sonst gleich guten mechanischen Festigkeiten und Brandverhalten.

### Vergleichsbeispiel 4: Herstellung eines Formkörpers aus Mica mit einem silikatischen Binder mit Modul 10 ohne Nachbehandlung

Zu weiteren Vergleichszwecken wird ein Formkörper aus Mica mit einem Binder mit Modul 10 ohne Nachbehandlung hergestellt.

60 Gewichtsteile Mica (Typ wie bei Beispiel 8) werden mit 25 Gewichtsteilen einer Lösung enthaltend ein erstes silikatisches Bindemittel gemäß Beispiel 7 (einer wässrigen Alkalisilikatlösung mit einem Modul von 10 und einem Feststoffgehalt von 33 Gew.-%) gemäß der Vorgehensweise in Beispiel 3 gemischt, gepresst, getrocknet. Das weitere Verfahren erfolgt gemäß Vergleichsbeispiel 1.

Es wird ein Formkörper (12 Gew.-% Binderanteil bezogen auf das Gesamtgewicht des Formkörpers bei sonst gleichen Maßen wie in Beispiel 8) mit schlechter mechanischer Festigkeit (4 kp) und sehr schlechter Wasserbeständigkeit (erste Partikelablösungen nach 1 Tag, vollständiger Zerfall nach 50 Tagen). Der Formkörper ist nicht brennbar und entwickelt keine Rauchgase.

Der erfindungsgemäß hergestellte Mica-Formkörper aus Beispiel 8 zeigt gegenüber dem nur einstufig ohne Nachbehandlung gebundenen Formkörper aus Vergleichsbeispiel 4 eine deutlich bessere mechanische Festigkeit und Wasserbeständigkeit.

## Patentansprüche

1. Verfahren zur Herstellung eines Formkörpers, **dadurch gekennzeichnet, dass** ein silikatischer Feststoff mit einer Lösung enthaltend ein erstes silikatisches Bindemittel vermischt und die Mischung dann verpresst wird und anschließend der gepresste Formkörper mit einer Lösung enthaltend ein zweites silikatisches Bindemittel nachbehandelt wird und das zweite silikatische Bindemittel einen höheren Modul als das erste silikatische Bindemittel aufweist.

2. Verfahren gemäß Anspruch 1, **dadurch gekennzeichnet, dass** als erstes silikatisches Bindemittel ein solches mit einem Modul von höchstens 50 eingesetzt wird.

3. Verfahren gemäß wenigstens einem der Ansprüche 1 oder 2, **dadurch gekennzeichnet, dass** als erstes silikatisches Bindemittel ein solches mit einem Modul von 1,5 bis 15 eingesetzt wird.

4. Verfahren gemäß wenigstens einem der Ansprüche 1 bis 3, **dadurch gekennzeichnet, dass** als zweites silikatisches Bindemittel ein solches mit einem Modul von mindestens 10 eingesetzt wird.

5. Verfahren gemäß wenigstens einem der Ansprüche 1 bis 4, **dadurch gekennzeichnet, dass** als zweites silikatisches Bindemittel ein solches mit einem Modul von 20 bis 1000 eingesetzt wird.

6. Verfahren gemäß wenigstens einem der Ansprüche 1 bis 5, **dadurch gekennzeichnet, dass** die Lösung enthaltend das erste silikatische Bindemittel einen Feststoffgehalt von 5 bis 60 Gew.-% aufweist.

7. Verfahren gemäß wenigstens einem der Ansprüche 1 bis 6, **dadurch gekennzeichnet, dass** die Lösung enthaltend das zweite silikatische Bindemittel einen Feststoffgehalt von 5 bis 60 Gew.-% aufweist.

8. Verfahren gemäß wenigstens einem der Ansprüche 1 bis 7, **dadurch gekennzeichnet, dass** die Nachbehandlung mit der Lösung enthaltend das zweite silikatische Bindemittel durch Tränken, Aufsprühen oder Aufstreichen und anschließende Trocknung erfolgt.

9. Verfahren gemäß wenigstens einem der Ansprüche 1 bis 8, **dadurch gekennzeichnet, dass** der silikatische Feststoff bevorzugt ein Schichtsilikat ist.

10. Verfahren gemäß wenigstens einem der Ansprüche 1 bis 9, **dadurch gekennzeichnet, dass** der silikatische Feststoff ein Vermiculit, Perlit oder Glimmer ist.

11. Verfahren gemäß wenigstens einem der Ansprüche 1 bis 10, **dadurch gekennzeichnet, dass** der silikatische Feststoff ein Vermiculit ist.

## Claims

1. Process for producing a moulding, **characterized in that** a silicatic solid is mixed with a solution comprising a first silicatic binder and the mixture is then press-moulded, and the compression moulding is then post-treated with a solution comprising a second silicatic binder and the second silicatic binder has a higher modulus than the first silicatic binder.

2. Process according to Claim 1, **characterized in that** the first silicatic binder used comprises one whose modulus is at most 50.

3. Process according to at least one of Claims 1 and 2, **characterized in that** the first silicatic binder used comprises one whose modulus is from 1.5 to 15.

4. Process according to at least one of Claims 1 to 3, **characterized in that** the second silicatic binder used comprises one whose modulus is at least 10.

5. Process according to at least one of Claims 1 to 4, **characterized in that** the second silicatic binder used comprises one whose modulus is from 20 to 1000.

6. Process according to at least one of Claims 1 to 5, **characterized in that** the solution comprising the first silicatic binder has a solids content of from 5 to 60% by weight.

7. Process according to at least one of Claims 1 to 6, **characterized in that** the solution comprising the second silicatic binder has a solids content of from 5 to 60% by weight.

8. Process according to at least one of Claims 1 to 7, **characterized in that** the post-treatment with the solution comprising the second silicatic binder takes place via soaking, spray-application or spread-application, and subsequent drying.

9. Process according to at least one of Claims 1 to 8, **characterized in that** the silicatic solid is preferably a phyllosilicate.

10. Process according to at least one of Claims 1 to 9, **characterized in that** the silicatic solid is a vermiculite, perlite, or mica.

11. Process according to at least one of Claims 1 to 10, **characterized in that** the silicatic solid is a vermiculite.

## Revendications

1. Procédé de fabrication d'un corps façonné, **caractérisé en ce qu'**on mélange une matière solide constituée de silicate avec une solution contenant un premier liant silicate et on comprime ensuite le mélange, puis on traite le corps façonné comprimé avec une solution contenant un deuxième liant silicate, ce deuxième liant présentant un module plus élevé que celui du premier liant.

2. Procédé selon la revendication 1, **caractérisé en ce qu'**on utilise, comme premier liant silicate, un liant d'un module maximum de 50.

3. Procédé selon au moins l'une des revendications 1 ou 2, **caractérisé en ce qu'**on utilise, comme premier liant silicate, un liant d'un module de 1,5 à 15.

4. Procédé selon au moins l'une quelconque des revendications 1 à 3, **caractérisé en ce qu'**on utilise, comme deuxième liant silicate, un liant d'un module d'au moins 10.

5. Procédé selon au moins l'une quelconque des revendications 1 à 4, **caractérisé en ce qu'**on utilise, comme deuxième liant silicate, un liant d'un module de 20 à 1000.

6. Procédé selon au moins l'une quelconque des revendications 1 à 5, **caractérisé en ce que** la solution contenant le premier liant silicate présente une teneur en matière solide de 5 à 60% en poids.

7. Procédé selon au moins l'une quelconque des revendications 1 à 6, **caractérisé en ce que** la solution contenant le deuxième liant silicate présente une teneur en matière solide de 5 à 60% en poids.

8. Procédé selon au moins l'une quelconque des revendications 1 à 7, **caractérisé en ce que** le post-traitement avec la solution contenant le deuxième liant silicate est effectué par imprégnation, pulvérisation ou application au pinceau, suivie d'un séchage.

9. Procédé selon au moins l'une quelconque des revendications 1 à 8, **caractérisé en ce que** la matière solide constituée de silicate est de préférence un silicate stratifié.

10. Procédé selon au moins l'une quelconque des revendications 1 à 9, **caractérisé en ce que** la matière solide constituée de silicate est une vermiculite, une perlite ou un mica.

11. Procédé selon au moins l'une quelconque des revendications 1 à 10, **caractérisé en ce que** la matière solide constituée de silicate est une vermiculite.
